# EUROPEAN PATENT APPLICATION

(11) **EP 4 170 337 A1**
(43) Date of publication of application: **26.04.2023**
(21) Application number: 21828256.4
(22) Date of filing: 13.04.2021
(51) Int. Cl.: G01N 27/407

(54) **HYDROGEN DETECTION SENSOR AND MANUFACTURING METHOD THEREFOR**

(30) Priority: 22.06.2020 KR 20200075466
(71) Applicant: Daehyunst Co., Ltd, Hwaseong-si, Gyeonggi-do 18578 (KR)
(72) Inventor: SEO, Hyung Tak, Seoul 06544 (KR); HAN, Seung Ik, Suwon-si Gyeonggi-do 16323 (KR)
(74) Representative: Laine IP Oy
(86) International application number: PCT/KR2021/004615
(87) International publication number: WO 2021/261723

(57) **Abstract**

A hydrogen detecting sensor is initiated. The hydrogen detecting sensor include a substrate; a heater layer formed on the substrate so as to generate heat; a sensing element formed on a top face of the heater layer, wherein the sensing element includes a sensing layer, wherein the sensing layer has a structure in which two or more alloy layers are stacked, wherein each of the two or more alloy layers is made of an alloy of a catalyst metal and a transition metal, wherein an electrical resistance of the catalyst metal reversibly changes when the catalyst metal adsorbs hydrogen, wherein a ratio of a content of the transition metal to a content of the catalyst metal in each of the two or more alloy layers continuously changes based on a vertical level metal in each of the two or more alloy layers, wherein the sensing element measures an electrical resistance based on a hydrogen concentration; and a compensation element formed on the top face of the heater layer so as to be spaced apart from the sensing element, wherein the compensation element includes: a material having the same structure as the structure of the sensing layer; and a protective layer covering the material layer so as to prevent an external substance from invading the material layer, wherein the compensation element measures an electrical resistance based on temperature change.

## Description

### FIELD

The present disclosure relates to a hydrogen detecting sensor capable of detecting hydrogen in an electrical manner and a method for manufacturing the same, wherein the hydrogen detecting sensor includes a temperature compensation element to separate a signal due to hydrogen response at room temperature and high temperature.

### DESCRIPTION OF RELATED ART

Recently, extensive research is being conducted to use hydrogen gas a renewable clean energy source that replaces petroleum. Hydrogen fuel is considered as a candidate for future energy because it has high heat of combustion and low ignition energy and burns completely. However, since hydrogen is highly volatile, flammable and explosive, it is dangerous when a concentration of hydrogen exceeds a critical value. On the other hand, since hydrogen is a flammable gas with no color, smell, or taste, it cannot be detected by human senses. Therefore, in order to safely use hydrogen as a fuel, a separate hydrogen sensor is essential.

Various types of hydrogen sensors have been reported, among which an electric hydrogen sensor is the most widely used. In particular, the electrical hydrogen sensor using palladium with high hydrogen adsorption capacity is widely used. In this regard, when palladium is exposed to low concentration hydrogen, palladium has an alpha (α) phase and thus electrical conductivity thereof changes in proportion to a hydrogen concentration. However, when palladium is exposed to high concentration hydrogen, the alpha (α) phase is converted to a beta (β) phase, and electrical conductivity of the beta (β) phase palladium does not change in proportion to the hydrogen concentration. Thus, the palladium cannot be used as a hydrogen detection material. Further, when palladium transitions from the alpha (α) phase to the beta (β) phase, the transition accompanies volume expansion. Thus, when palladium is repeatedly exposed to the high-concentration hydrogen, cracks and fractures occur in a hydrogen detection layer, resulting in a problem of not detecting the hydrogen. Therefore, the conventional electrical hydrogen sensor using palladium as a hydrogen detecting material could only detect hydrogen at a concentration of lower than about 4%.

In order to solve the problem of the transition of palladium into the beta (β) phase, a scheme of alloying palladium with a different metal to prepare an alloy and using the alloy as a hydrogen detection material has been proposed. Specifically, a scheme of alloying palladium with a different metal via simultaneous deposition of palladium and the different metal using sputtering has been proposed. However, using this scheme, there is a problem in that a ratio of contents of palladium and the different metal is non-uniform due to interference between plasmas of the two materials, and thus an alloy layer of a uniform content ratio could not be repeatedly formed. In addition, there is a problem in that a degree of freedom to improve characteristics of the hydrogen sensing material is low due to a limited deposition condition, and two or more deposition guns are essentially required for the simultaneous deposition.

Further, the conventional hydrogen sensor has a problem in that an electrical resistance value changes in a changing temperature environment, thus making it difficult to separate only a signal due to hydrogen response, such that reliability of hydrogen detection is deteriorated.

### DISCLOSURE

### TECHNICAL PURPOSE

One purpose of the present disclosure is to provide a hydrogen detecting sensor including a sensing element including a sensing layer made of an alloy of a catalyst metal and a transition metal and having a content of the transition metal varying depending on a position therein, and a compensation element for measuring an electrical resistance according to temperature change such that the sensor stably detects high concentration hydrogen.

Another purpose of the present disclosure is to provide a method for manufacturing the hydrogen detecting sensor.

### TECHNICAL SOLUTION

A first aspect of the present disclosure provides a hydrogen detecting sensor comprising: a substrate; a heater layer formed on the substrate so as to generate heat; a sensing element formed on a top face of the heater layer, wherein the sensing element includes a sensing layer, wherein the sensing layer has a structure in which two or more alloy layers are stacked, wherein each of the two or more alloy layers is made of an alloy of a catalyst metal and a transition metal, wherein an electrical resistance of the catalyst metal reversibly changes when the catalyst metal adsorbs hydrogen, wherein a ratio of a content of the transition metal to a content of the catalyst metal in each of the two or more alloy layers continuously changes based on a vertical level metal in each of the two or more alloy layers, wherein the sensing element measures an electrical resistance based on a hydrogen concentration; and a compensation element formed on the top face of the heater layer so as to be spaced apart from the sensing element, wherein the compensation element includes: a material having the same structure as the structure of the sensing layer; and a protective layer covering the material layer so as to prevent an external substance from invading the material layer, wherein the compensation element measures an electrical resistance based on temperature change.

In one implementation of the hydrogen detecting sensor, the sensing element further includes: first and second electrodes in contact with the sensing layer and spaced apart from each other; and an analysis circuit electrically connected to the first and second electrodes so as to measure change in the electrical resistance of the sensing layer based on the hydrogen concentration.

In one implementation of the hydrogen detecting sensor, the catalyst metal includes palladium or platinum, and the transition metal includes nickel or magnesium.

In one implementation of the hydrogen detecting sensor, in each of the alloy layers, the ratio of the content of the transition metal to the content of the catalyst metal in an area adjacent to each of a top face and a bottom face of each alloy layer is lower than the ratio at a vertical middle level between the top face and the bottom face.

In one implementation of the hydrogen detecting sensor, the ratio of the content of the transition metal to the content of the catalyst metal is highest at the vertical middle level in each of the alloy layers, wherein the ratio gradually decreases as each of the alloy layers extends toward each of the top face and the bottom face.

In one implementation of the hydrogen detecting sensor, the heater layer is made of platinum (Pt), and is adhered to the substrate via an adhesive layer including titanium (Ti) or chromium (Cr).

In one implementation of the hydrogen detecting sensor, the compensation element further includes: third and fourth electrodes in contact with the material layer coated with the protective layer and spaced apart from each other; and an analysis circuit electrically connected to the third and fourth electrodes so as to measure change in the electrical resistance of the material layer based on the temperature change.

In one implementation of the hydrogen detecting sensor, the protective layer includes PTFE (polytetrafluoroethylene), PDMS (polydimethylsiloxane) or aluminum oxide (Al₂O₃).

A second aspect of the present disclosure provides a method for manufacturing a hydrogen detecting sensor, the method comprising: depositing platinum on a substrate to form a heater layer; disposing a plurality of transition metal layers and a plurality of catalyst metal layers on the top face of the heater layer such that the plurality of transition metal layers and the plurality of catalyst metal layers are alternately stacked on top of each other, wherein each of the catalyst metal layers is made of a catalyst metal whose an electrical resistance reversibly changes when the catalyst metal adsorbs hydrogen, wherein each of the plurality of transition metal layers is made of a transition metal suppressing phase transformation of the catalyst metal; diffusing the transition metal into the catalyst metal layers to alloy the catalyst metal with the transition metal to form a hydrogen sensing layer made of the alloy, thereby manufacturing a sensing element including the sensing layer; forming a material layer on the top face of the heater layer in the same manner as the formation manner of the hydrogen sensing layer, wherein the material layer is spaced apart from the hydrogen sensing layer; and forming a protective layer covering an exposed surface of the material layer, thereby manufacturing a compensation element including the material layer and the protective layer.

In one implementation of the method, each of the catalyst metal layers is formed by performing a sputtering process of palladium or platinum, wherein each of the transition metal layers is formed by performing a sputtering process of nickel or magnesium.

In one implementation of the method, each of the catalyst metal layers is formed to have a thickness in a range of 1 nm to 4 nm, wherein each of the transition metal layers is formed to have a thickness of 0.1 to 0.5 times of the thickness of the catalyst metal layer.

In one implementation of the method, each of the transition metal layers is formed to have a thickness of 0.1 to 0.3 times of the thickness of the catalyst metal layer.

In one implementation of the method, the heater layer is formed by performing an e-beam deposition process of platinum, wherein the heater layer is adhered to the substrate via an adhesive layer including titanium (Ti) or chromium (Cr).

In one implementation of the method, the protective layer is formed by performing an atomic layer deposition (ALD) process of aluminum oxide (Al₂O₃).

In one implementation of the method, the protective layer is formed by performing a deposition process or sputtering process of polytetrafluoroethylene (PTFE) or polydimethylsiloxane (PDMS).

### TECHNICAL EFFECT

According to the hydrogen detecting sensor and the method for manufacturing the same according to an embodiment of the present disclosure, the plurality of catalyst metal layers and the plurality of transition metal layers are alternately stacked on top of each other, and the transition metal of the transition metal layers is diffused into the catalyst metal layers to form the sensing layer for detecting the hydrogen. Thus, the plasma interference occurring during the formation of the alloy layer via the simultaneous deposition of the different metals in a conventional manner may be prevented, and thus, the alloy layer of the uniform composition may be repeatedly formed, and as a result, mass productivity may be improved. In addition, the catalyst metal layer and the transition metal layer are formed independently, such that the degree of freedom in improving material properties may be greatly increased.

Further, the hydrogen detecting sensor includes the compensation element whose the electrical resistance changes only based on the temperature. The compensation element may correct the resistance characteristics of the sensing element in a changing temperature environment. Thus, the sensor is able to separate a signal caused by hydrogen response at room temperature and high temperature.

In addition, the heater layer may be used to control the baseline as a reference point of a signal at a low temperature operation and a temperature higher than room temperature, thereby achieving an effect of securing a very wide operating temperature.

### BRIEF DESCRIPTION OF DRAWINGS

FIG. 1A and FIG. 1B are diagrams for illustrating a hydrogen detecting sensor according to an embodiment of the present disclosure.
FIG. 2 is a cross-sectional view for illustrating a sensing layer as shown in FIG. 1.
FIG. 3 is a plan view of one embodiment of a sensing element as shown in FIG. 1.
FIG. 4 is a perspective view of one embodiment of a compensation element as shown in FIG. 1.
FIG. 5A is a graph showing hydrogen detection characteristics of a first hydrogen detecting sensor including a sensing layer of a structure in which two alloy layers as described above are stacked. FIG. 5B is a graph showing hydrogen detection characteristics of a second hydrogen detecting sensor including a sensing layer composed of only one alloy layer.
FIGS. 6A and 6B are graphs showing color change characteristics of each of a compensation element (Present Example) to which a PDMS protective layer is applied and a compensation element (Comparative Example) identical therewith except for the PDMS protective layer.
FIG. 7 is a graph showing hydrogen detection characteristics of each of a sensing element and a compensation element to which an Al₂O₃ protective layer is applied, according to an embodiment of the present disclosure.
FIG. 8 shows a result of durability test evaluation of a hydrogen detecting sensor according to an embodiment of the present disclosure.
FIG. 9 is a cross-sectional view for illustrating a method of forming a sensing layer according to an embodiment of the present disclosure.
FIG. 10 is a graph to illustrate a sensitivity of a sensing layer based on a ratio of a thickness of a transition metal layer to a 3 nm thickness of a catalyst metal layer.

### DETAILED DESCRIPTIONS

Hereinafter, an embodiment of the present disclosure will be described in detail with reference to the accompanying drawings. The present disclosure may be variously modified and may take many forms. Thus, specific embodiments will be illustrated in the drawings and described in detail herein. However, the specific embodiments are not intended to limit the present disclosure thereto. It should be understood that all changes, equivalents thereto, or substitutes therewith are included in a scope and spirit of the present disclosure. In describing the drawings, similar reference numerals are used for similar components. In the accompanying drawings, a dimension of each of structures is enlarged to a value than an actual value for clarity of illustration of the present disclosure.

It will be understood that, although the terms "first", "second", "third", and so on may be used herein to describe various elements, components, regions, layers and/or sections, these elements, components, regions, layers and/or sections should not be limited by these terms. These terms are used to distinguish one element, component, region, layer or section from another element, component, region, layer or section. Thus, a first element, component, region, layer or section described below could be termed a second element, component, region, layer or section, without departing from the spirit and scope of the present disclosure.

The terminology used herein is for the purpose of describing particular embodiments only and is not intended to limit the present disclosure. As used herein, the singular forms "a" and "an" are intended to include the plural forms as well, unless the context clearly indicates otherwise. It will be further understood that the terms "comprises", "comprising", "includes", and "including" when used in this specification, specify the presence of the stated features, integers, operations, elements, and/or components, but do not preclude the presence or addition of one or more other features, integers, operations, elements, components, and/or portions thereof.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

### <Hydrogen detecting sensor>

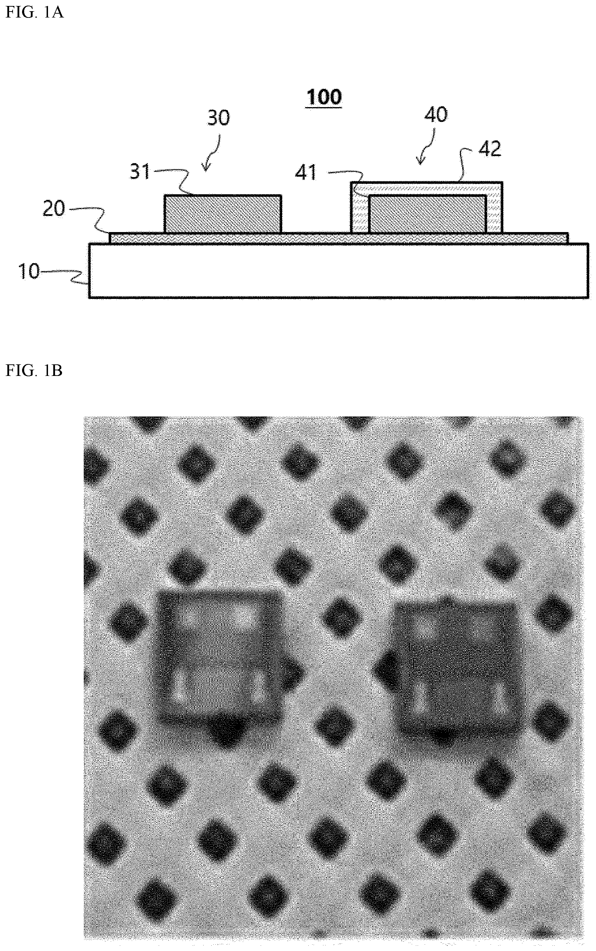

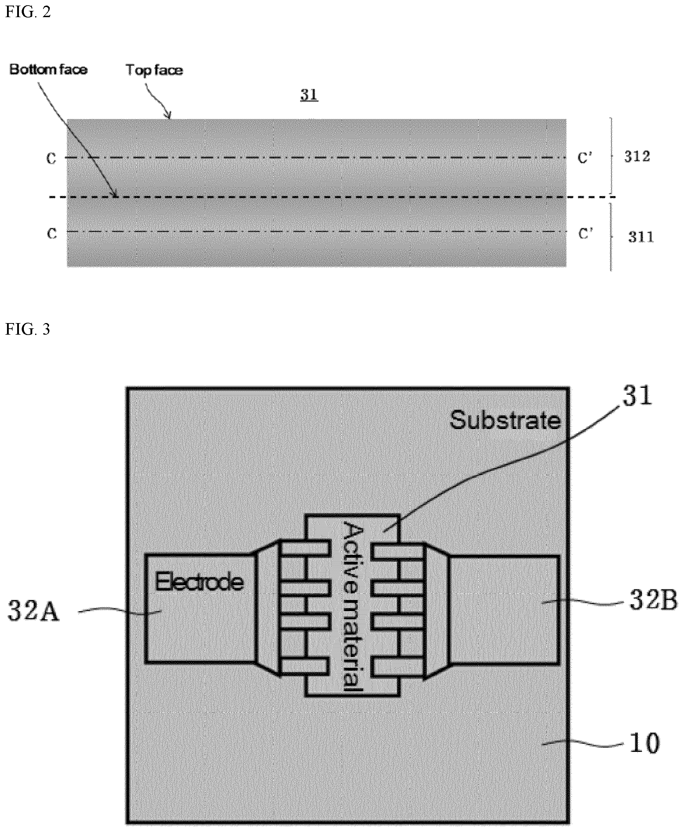

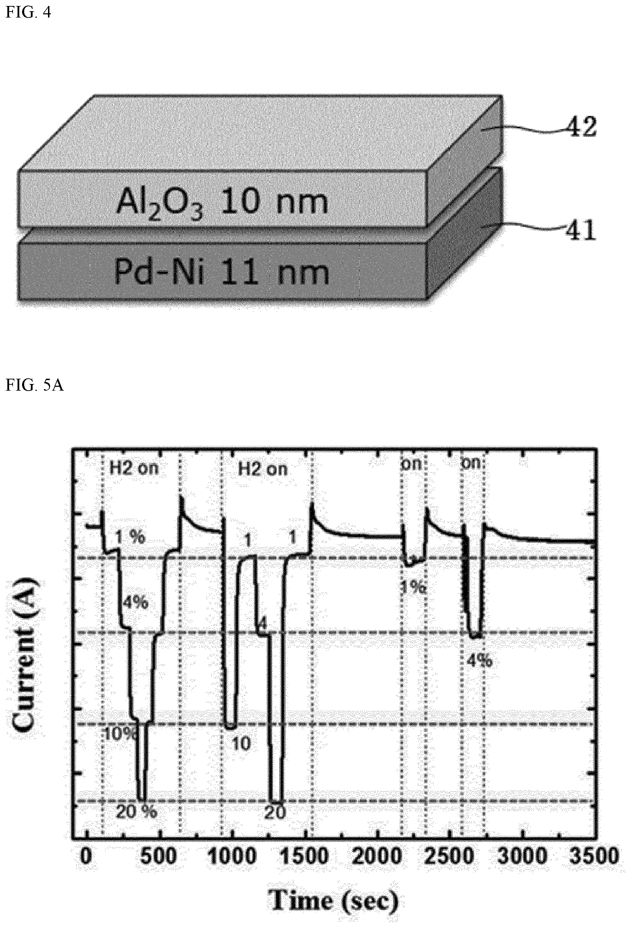

FIGS. 1A and 1B are diagrams for illustrating a hydrogen detecting sensor according to an embodiment of the present disclosure. FIG. 2 is a cross-sectional view for illustrating a sensing layer as shown in FIG. 1. FIG. 3 is a plan view of one embodiment of a sensing element as shown in FIG. 1. FIG. 4 is a perspective view of one embodiment of a compensation element as shown in FIG. 1.

Referring to FIG. 1 to FIG. 4, a hydrogen detecting sensor 100 according to an embodiment of the present disclosure may include a substrate 10, a heater layer 20, a sensing element 30 and a compensation element 40.

The substrate 10 may be embodied as a substrate of each of various materials and each of structures, and is not particularly limited. For example, the substrate 10 may be embodied as a substrate made of paper, polymer, glass, metal, ceramic, or the like.

The heater layer 20 is formed on the substrate 10 and generates heat to increase activity of a hydrogen sensing material, and a baseline as a reference point of a signal at a low temperature operation and at a temperature above a room temperature. The heater layer 20 may be made of a material with little chemical change even when being heated to a high temperature. For example, the heater layer 20 may be made of platinum (Pt). Further, the heater layer 20 may be adhered to the substrate 10 via an adhesive layer (not shown) including titanium (Ti) or chrome (Cr). In one embodiment, a ratio of a thickness of the heater layer 20 (platinum) and a thickness of the adhesive layer (titanium) may be 10:1: In one example, the heater layer 20 may be made of platinum (200 nm thick) and the adhesive layer may be made of titanium (20 nm thick). However, the present disclosure is not limited thereto.

The sensing element 30 is formed on a top face of the heater layer 20 so as to measure an electrical resistance based on a hydrogen concentration and includes a sensing layer 31.

The sensing layer 31 may be positioned on the top face of the heater layer 20 and may be made of a material capable of sensing hydrogen. In one embodiment, the sensing layer 31 may be made of a material which can adsorb hydrogen, and whose an electrical resistance reversibly changes when adsorbing the hydrogen. For example, the sensing layer 31 may be made of an alloy of a transition metal and a noble metal catalyst metal whose an electrical resistance increases as an amount of adsorption of hydrogen thereof increases. In this case, the catalyst metal may include palladium (Pd), platinum (Pt), and the like, and the transition metal may include nickel (Ni) and magnesium (Mg), which may suppress transformation of a crystal phase of the catalyst metal.

In one embodiment, the sensing layer 31 may be made of an alloy of palladium and nickel. Pure palladium has a strong ability to adsorb hydrogen, and an alpha (α) phase of the crystal phase of palladium may reversibly change in an electrical resistance thereof in proportion to an amount of adsorption of hydrogen thereof. Thus, palladium is generally used as a hydrogen detection material. However, when the adsorption amount of hydrogen thereof is excessively increased, that is, when being exposed to high concentration hydrogen of 4% or higher, there is a problem in that the crystal phase thereof is transformed from the alpha (α) phase into a beta (β) phase. Palladium of the beta (β) phase has a characteristic that an electrical resistance thereof converges to a constant value when the adsorption amount of hydrogen thereof increases. Thus, palladium of the beta (β) phase cannot be used as a hydrogen detection material. Further, when the alpha (α) phase is transformed into the beta (β) phase, a volume expansion occurs, such that cracks or fractures occur therein due to a reversible reaction with hydrogen. Thus, durability and lifespan of the sensing layer 31 are deteriorated.

However, when palladium of the alpha (α) phase is alloyed with nickel or magnesium, the phase transformation may be suppressed and thus the transformation of the alpha (α) phase into the beta (β) phase may be prevented even when being exposed to high concentration hydrogen. As a result, when the alloy of palladium and nickel is used as a hydrogen detection material, the alloy can detect the high concentration hydrogen.

In one embodiment of the present disclosure, the sensing layer 31 may have a stack structure in which two or more alloy layers 311 and 312 are stacked, wherein each of the two or more alloy layers may be made of an alloy of the catalyst metal and the transition metal, and a ratio of a content of the transition metal with respect to a content of the catalyst metal continuously changes depending on a position in each of the two or more alloy layers. In each of the two or more alloy layers 311 and 312, the content of the transition metal in an area adjacent to a top face and a bottom face of each of the two or more alloy layers 311 and 312 may be the lowest, while the content of the transition metal may be highest in a vertical middle area between the top face and the bottom face. That is, in each of the alloy layers 311 and 312, the ratio of the content of the transition metal with respect to the content of the catalyst metal may be highest in the vertical middle area. The ratio may gradually decrease as each of the alloy layers 311 and 312 extends toward each of the top face and the bottom face.

In one example, it is preferable that an area composed only of the catalyst metal or an area composed only of the transition metal is not formed inside each of the alloy layers 311 and 312. When an area composed only of the catalyst metal is formed, the phase transformation of the catalyst metal may occur when the area is exposed to high concentration hydrogen. When an area composed only of the transition metal is formed, the transition metal traps hydrogen and delays hydrogen diffusion, so that a response speed of the sensing layer 31 related to detection of the hydrogen may be significantly lowered.

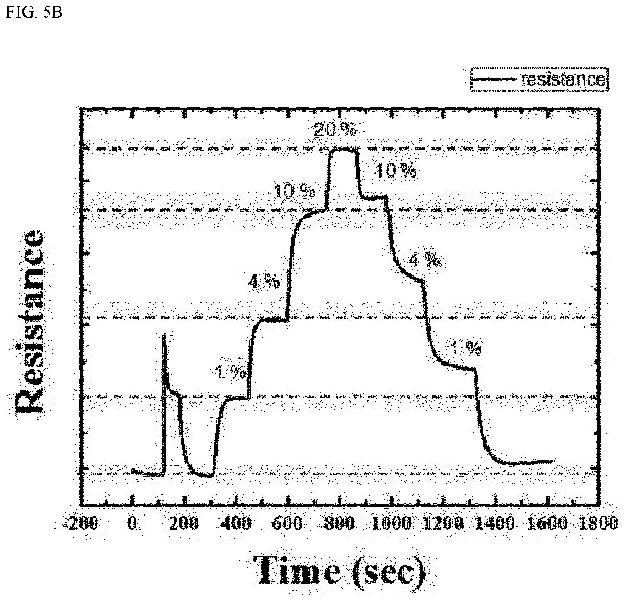

In one embodiment, in order to improve reversibility of the reaction of the sensing layer 31 with hydrogen to improve a lifespan of the hydrogen detecting sensor, the sensing layer 31 may have a structure in which the two or more alloy layers 311 and 312 are stacked. When the sensing layer 120 is composed of only one alloy layer, the reversibility of the reaction of the sensing layer with hydrogen is deteriorated in a short time, which may cause a problem in that the life of the sensor is shortened. This will be described later with reference to FIG. 5A and FIG. 5B. When a thickness of the sensing layer 31 exceeds 50 nm, a possibility of internal incomplete alloying increases. Thus, cracks may occur when the sensing layer is repeatedly exposed to high concentration hydrogen. Therefore, it is preferable that the number of the alloy layers included in the sensing layer 31 is about 10 or smaller.

In one example, the sensing element 30 may include not only the sensing layer 31 but also first and second electrodes 32Aand 32B and an analysis circuit 33.

The first and second electrodes 32A and 32B may be spaced apart from each other and may be disposed on a top face of the heater layer 20 and may contact the sensing layer 31. Each of the first and second electrodes 32A and 32B may be made of a conductive material, for example, metal. A shape or a structure thereof is not particularly limited.

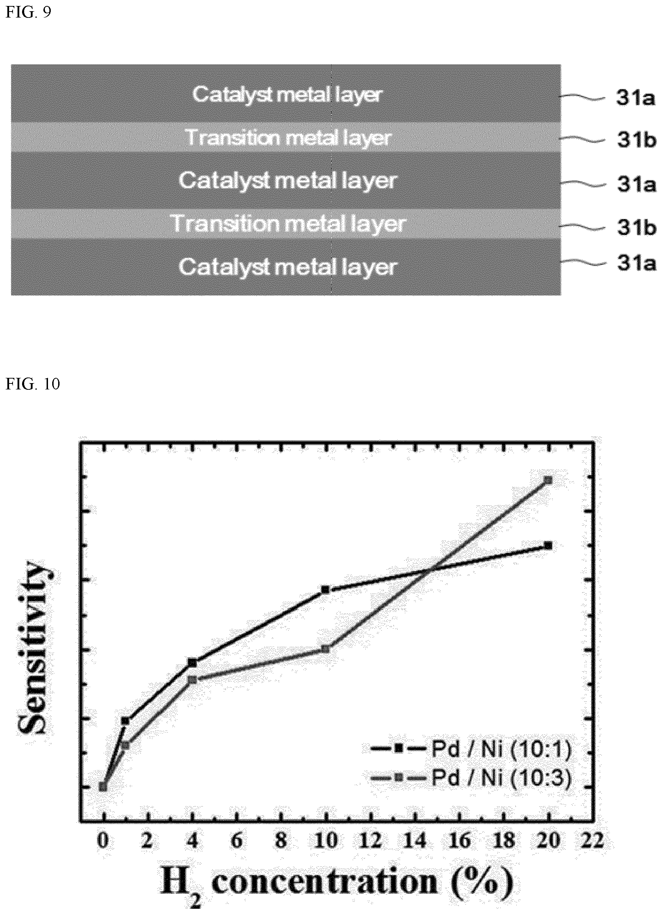

In one embodiment, in order to more accurately and immediately measure change in a resistance value of the sensing layer 31, each of the first and second electrodes 32A and 32B may have a structure in which each of the first and second electrodes 32A and 32B is in contact with the sensing layer 31 at a plurality of contact areas shown in FIG. 3. In another embodiment, each of the first and second electrodes 32A and 32B may be made of the catalyst metal. For example, as shown in FIG. 9, a plurality of catalyst metal layers 31a and a plurality of transition metal layers 31b may be stacked alternately with each other, and then the transition metal and the catalyst metal may diffuse into each other. Thus, the sensing layer 31 may be formed. In this case, each of the first and second electrodes 32A and 32B may be formed from at least one of the catalyst metal layers 31a. Specifically, the transition metal layers 31b and the catalyst metal layers 31a may be alternately stacked on top of each other such that both opposing edge portions of each of the catalyst metal layers 31a are exposed. Then, the transition metal of each of the transition metal layers 31b may be diffused into each of the catalyst metal layers 31a. At this time, the both exposed edge portions of each of the catalyst metal layers 31a may act as the first and second electrodes 32A and 32B, respectively. In this case, a separate process for forming the first and second electrodes 32A and 32B is not required, such that a manufacturing cost of the hydrogen detecting sensor 100 according to an embodiment of the present disclosure may be reduced.

The analysis circuit 33 may be electrically connected to the first electrode 32A and the second electrode 32B so as to measure a change in the electrical resistance of the sensing layer 31. A configuration of the analysis circuit 33 is not particularly limited as long as the analysis circuit 33 can measure the change in the electrical resistance of the sensing layer 31.

The compensation element 40 may be formed on the top face of the heater layer 20 so as to be spaced apart from the sensing element 30. An electrical resistance of the compensation element 40 may change only based on a varying temperature. Thus, the compensation element 40 may serve to correct resistance characteristics of the sensing element 30 in a changing temperature environment. Therefore, unlike the sensing element 30, the compensation element 40 should not be exposed to an external substance. In particular, a reaction of the compensation element 40 with hydrogen should be minimized. Accordingly, the compensation element 40 may include a material layer 41 and a protective layer 42 covering the material layer 42 so as to prevent the external substance from invading into the material layer 42.

The material layer 41 may be positioned on the top face of the heater layer 20 so as to be spaced apart from the sensing layer 31. Since the material layer 41 has the same material and the same structure as those of the sensing layer 31, description thereof will be omitted.

The protective layer 42 may cover an exposed surface of the material layer 41 so as to prevent the external substance from invading into the material layer 42. Thus, the protective layer 42 may be made of a polymeric material or aluminum oxide (Al₂O₃) capable of preventing the external substance from invading into the material layer 42. For example, the protective layer 42 may be made of polytetrafluoroethylene (PTFE), polydimethylsiloxane (PDMS), or aluminum oxide (Al₂O₃).

In one embodiment, the PDMS may be used in various forms. The PDMS may be preferably used in a liquid adhesive form. Further, aluminum oxide (Al₂O₃) may be deposited on the material layer 41 via an atomic layer deposition process to form the protective layer 42.

In one example, the compensation element 40 may further include third and fourth electrodes 43A and 43B and an analysis circuit 44 (which are not shown in the drawings).

The third and fourth electrodes 43A and 43B may be spaced apart from each other and may be disposed on the top face of the heater layer 20 and may contact the material layer 41 coated with the protective layer 42. A material composition, a shape and a structure of each of the third and fourth electrodes 43A and 43B may be the same as those of each of the first and second electrodes 32A and 32B as described above. Thus, a description thereof will be omitted.

The analysis circuit 44 may be electrically connected to the third electrode 43A and the fourth electrode 43B so as to measure a change in an electrical resistance of the material layer 41 according to change in a temperature. A configuration of the analysis circuit 44 is not particularly limited as long as the analysis circuit 44 can measure the change in the electrical resistance according to the change in a temperature.

FIG. 5A is a graph showing hydrogen detection characteristics of a first hydrogen detecting sensor including a sensing layer of a structure in which two alloy layers as described above are stacked. FIG. 5B is a graph showing hydrogen detection characteristics of a second hydrogen detecting sensor including a sensing layer composed of only one alloy layer. The first hydrogen detecting sensor and the second hydrogen detecting sensor have the same configuration except for a configuration of the sensing layer.

First, referring to FIG. 5A, it may be identified that even when the sensing layer of the first hydrogen detecting sensor is exposed to hydrogen at each of various concentrations such that adsorption and desorption reactions of hydrogen by and from the sensing layer are repeatedly induced, a resistance value of the sensing layer of the first hydrogen detecting sensor under a specific hydrogen concentration is maintained to be constant. Specifically, as clearly as shown in FIG. 5A, it may be identified that the resistance value of the sensing layer corresponding to each of the hydrogen concentrations of 0%, 1%, 4%, 10%, and 20% is maintained to be constant over time.

On the contrary, referring to FIG. 5B, it may be identified that even when the sensing layer of the second hydrogen detecting sensor is exposed to hydrogen at each of various concentrations such that adsorption and desorption reactions of hydrogen by and from the sensing layer are repeatedly induced, a resistance value of the sensing layer of the second hydrogen detecting sensor under a specific hydrogen concentration is not maintained to be constant. Specifically, it may be identified that the resistance value of the sensing layer at about 400 seconds and the resistance value thereof at about 1200 seconds under a hydrogen concentration of 1% are different from each other; The resistance value at about 500 seconds and the resistance value at about 1000 seconds of the sensing layer under a hydrogen concentration of 4% are different from each other; and the resistance value at about 700 seconds and the resistance value at about 900 seconds of the sensing layer under a hydrogen concentration of 10% are different from each other.

Thus, in an embodiment of the present disclosure, in order to maintain the reversibility of the sensing layer for a long time to improve the lifetime of the hydrogen detecting sensor, it is preferable that the sensing layer has the structure in which the two or more alloy layers as described above are stacked.

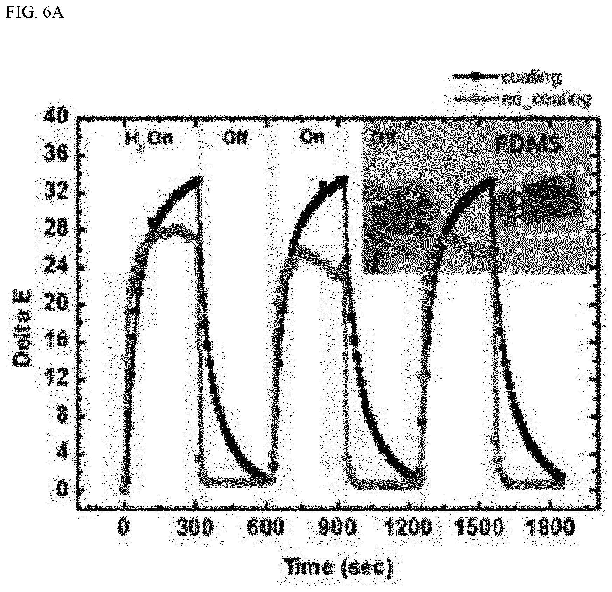

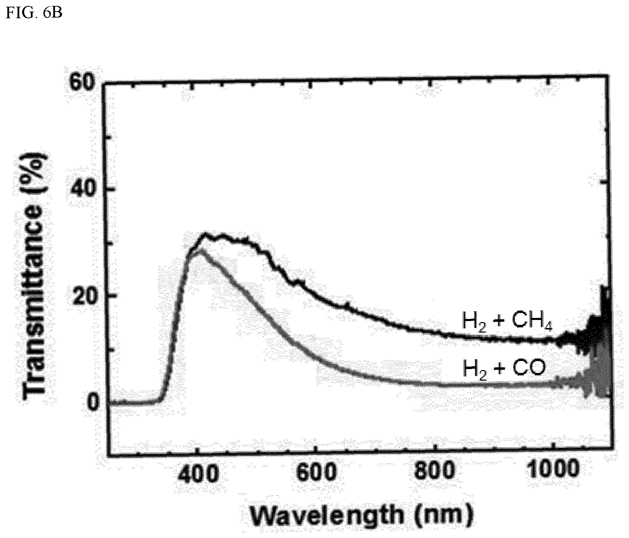

FIGS. 6A and 6B are graphs showing color change characteristics of each of a compensation element (Present Example) to which a PDMS protective layer is applied and a compensation element (Comparative Example) identical therewith except for the PDMS protective layer.

First, referring to FIG. 6A, it may be identified that the compensation element to which the PDMS protective layer is applied has a color change rate (delta E) lower than that in Comparative Example. As shown in a top inset, it may be identified that the compensation element free of the PDMS protective layer has a distinct color change after 3 months.

Further, referring to FIG. 6B, it may be identified that the compensation element free of the PDMS protective layer exhibits high invasion of hydrogen into the compensation element despite presence of CO gas, resulting in high color change.

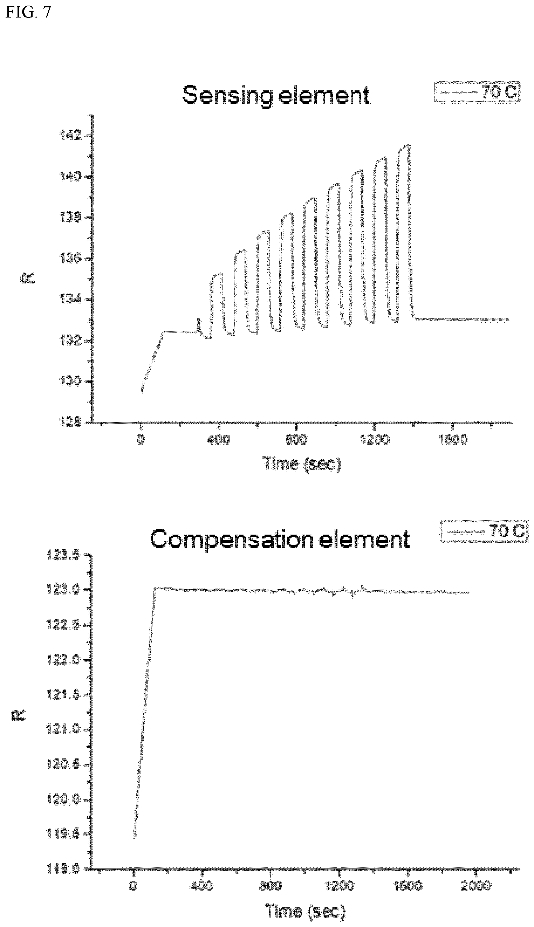

FIG. 7 is a graph showing hydrogen detection characteristics of each of a sensing element and a compensation element to which an Al₂O₃ protective layer is applied, according to an embodiment of the present disclosure.

Referring to FIG. 7, the sensing layer of the sensing element is exposed to hydrogen at each of various concentrations. At this time, as the hydrogen concentration increases, the resistance value of the sensing layer increases. However, the compensation element exhibits a negligible amount in terms of the change in the resistance value (the compensation element exhibits a reaction amount to hydrogen smaller than 10% of that of the sensing element).

Therefore, the compensation element according to the present disclosure may have the characteristic that the resistance value changes only based on the temperature and thus may correct the resistance characteristics of the sensing element in a temperature environment that changes.

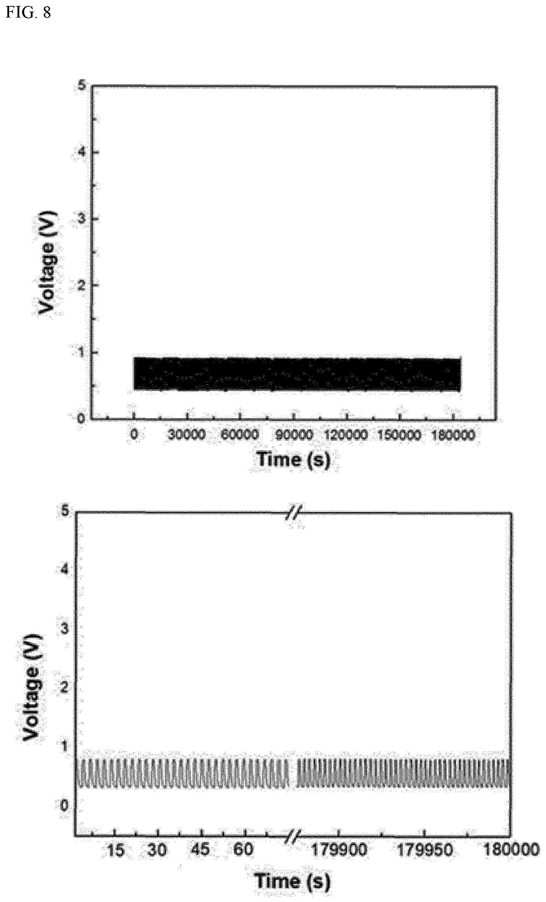

FIG. 8 shows a result of the durability test evaluation of the hydrogen detecting sensor according to an embodiment of the present disclosure. In this regard, in the durability test, a test gas is injected at a concentration of 10 vol%, and a hydrogen flow rate is maintained at 2 lpm, and then exposure to hydrogen is repeated 30,000 times (exposure frequency: 3 seconds On/3 seconds Off). Then, hydrogen detection sensitivity and zero-point stability are evaluated.

As shown in FIG. 8, an initial signal value is 0.876 V. After repeated evaluation, a signal value is 0.923 V, resulting in a maximum error percentage of 2.56 %.

That is, the hydrogen detecting sensor according to an embodiment of the present disclosure may have following evaluation results: an operating temperature -30 °C to 95 °C, a response time 1.9 sec, a sensor accuracy ± 5%, an operating humidity 10 to 90 RH, and durability of 5 years.

### <Method for manufacturing hydrogen detecting sensor>

A method for manufacturing a hydrogen detecting sensor according to an embodiment of the present disclosure includes depositing platinum on a substrate 10 to form the heater layer 20 (S110); disposing the plurality of catalyst metal layers 31a and the plurality of transition metal layers 31b on the top face of the heater layer 20 such that the plurality of catalyst metal layers 31a and the plurality of transition metal layers 31b are alternately stacked on top of each other via a physical vapor deposition process and diffusing the transition metal of the transition metal layer 31b to alloy the catalyst metal with the transition metal to form the hydrogen sensing layer 31 to manufacture the sensing element 30 (S120); and forming the material layer 41 on the top face of the heater layer 20 in the same manner as the formation manner of the hydrogen sensing layer 31 so as to be spaced apart from the hydrogen sensing layer 31, and forming the protective layer 42 covering an exposed surface of the material layer 41 to manufacture the compensation element 40 (S130).

In an embodiment, the heater layer 20 may be formed through an e-beam deposition process, and may be adhered to the substrate 10 via an adhesive layer (not shown) including titanium (Ti) or chromium (Cr). However, the present disclosure is not limited thereto.

In one embodiment, each of the catalyst metal layer 31a and the transition metal layer 31b may be formed through a sputtering process. The catalyst metal layer 31a may be made of a noble metal having excellent adsorption capacity of hydrogen, for example, palladium, platinum, etc. The transition metal layer 31b may be made of a transition metal capable of suppressing the phase transformation of the palladium or platinum, for example, nickel or magnesium.

Further, referring to FIG. 2 and FIG. 9, in the step of manufacturing the sensing element 30 (S200), the alloy layers 311 and 312 of the catalyst metal and the transition metal may be formed while the transition metal is diffused. In this case, since the catalyst metal of the catalyst metal layer 31a and the transition metal of the transition metal layer 31b are diffused to form the alloy layers 311 and 312, the ratio of the content of the transition metal relative to the content of the catalyst metal in each of the alloy layers 311 and 312 may decrease as each of the alloy layers 311 and 312 extends toward each of the top face and the bottom face thereof.

In one example, in one embodiment, in order to diffuse the catalyst metal of the catalyst metal layer 31a and the transition metal of the transition metal layer 31b to each other, heat treatment at a constant temperature may be performed on the catalyst metal layers 31a and the transition metal layers 31b which are alternately stacked with each other.

In one embodiment of the present disclosure, each of the catalyst metal layers 31a may be formed to have a thickness of about 4 nm or smaller, and each of the transition metal layers 31b may be formed to have a thickness of about 2 nm or smaller. When the thickness of the catalyst metal layer 31a exceeds 4 nm, the transition metal is not diffused into the catalyst metal layer 31a and thus is not alloyed with the catalyst metal such that an area composed of only the catalyst metal is formed. When the thickness of the transition metal layer 31b exceeds 2 nm, the transition metal of the transition metal layer 31b is not alloyed with the catalyst metal such that an area composed only of the transition metal is formed.

In one embodiment, the catalyst metal layer 31a may be formed to have a thickness of about 1 to 4 nm, and the transition metal layer 31b may be formed to have a thickness of about 0.1 to 0.5 times of the thickness of the catalyst metal layer 31a. When a ratio of the thickness of the transition metal layer 31b to the thickness of the catalyst metal layer 31a is smaller than 0.1, the effect of suppressing the phase transformation by the transition metal may be lowered. When the ratio exceeds 0.5, an area composed of only the transition metal remains or the content of the transition metal is excessively high across an entirety of the alloy layer, which delays hydrogen diffusion, resulting in a decrease in the response speed related to the hydrogen detection. In one example, the transition metal layer 31b is preferably formed to have a thickness of about 0.1 to 0.3 times of the thickness of the catalyst metal layer 31a. This will be described in detail with reference to FIG. 10.

FIG. 10 is a graph to illustrate the sensitivity of the sensing layer based on the ratio of the thickness of the transition metal layer to a 3 nm thickness of the catalyst metal layer. In FIG. 10, a black curve and a red curve are graphs measuring the sensitivity based on the hydrogen concentration of the sensing layer in which the ratio of the thickness of the catalyst metal layer and the thickness of the transition metal layer is 10:1 and 10:3, respectively.

Referring to FIG. 10, the sensing layer formed by alternately stacking two catalyst metal layers and two transition metal layers at a thickness ratio of 10:1, and diffusing the transition metal into the catalyst metal layer has small change in sensitivity based on the hydrogen concentration under a hydrogen concentration of 10% or higher. On the contrary, the sensing layer formed by alternately stacking two catalyst metal layers and two transition metal layers at a thickness ratio of 10:3, and diffusing the transition metal into the catalyst metal layer has large change in sensitivity based on the hydrogen concentration under a hydrogen concentration of 10% or higher and thus exhibits good linearity of the sensitivity. Therefore, in order to detect high concentration hydrogen, it is preferable that the ratio of the thickness of the catalyst metal layer and the thickness of the transition metal layer is in range of about 10:1 to about 10:3.

In one example, in one embodiment of the present disclosure, in order to maintain the reversibility of the reaction of the sensing layer 31 with the hydrogen for a long time, two or more transition metal layers 31b are preferably formed. This has been described in detail with reference to FIG. 5A and FIG. 5B, and thus duplicate description thereof is omitted.

In one embodiment, the protective layer 42 may be formed through a deposition process or sputtering process of polytetrafluoroethylene (PTFE) or polydimethylsiloxane (PDMS). Further, the PDMS may cover the exposed surface of the material layer 41 in a form of liquid adhesive.

In another embodiment, the protective layer 42 may be formed through an atomic layer deposition (ALD) process of aluminum oxide (Al₂O₃).

According to the hydrogen detecting sensor and the method for manufacturing the same according to an embodiment of the present disclosure, the plurality of catalyst metal layers and the plurality of transition metal layers are alternately stacked on top of each other, and the transition metal of the transition metal layers is diffused into the catalyst metal layers to form the sensing layer for detecting the hydrogen. Thus, the plasma interference occurring during the formation of the alloy layer via the simultaneous deposition of the different metals in a conventional manner may be prevented, and thus, the alloy layer of the uniform composition may be repeatedly formed, and as a result, mass productivity may be improved. In addition, the catalyst metal layer and the transition metal layer are formed independently, such that the degree of freedom in improving material properties may be greatly increased.

Further, the hydrogen detecting sensor includes the compensation element whose the electrical resistance changes only based on the temperature. The compensation element may correct the resistance characteristics of the sensing element in a changing temperature environment. Thus, the sensor is able to separate a signal caused by hydrogen response at room temperature and high temperature.

In addition, the heater layer may be used to control the baseline as a reference point of a signal at a low temperature operation and a temperature higher than room temperature, thereby achieving an effect of securing a very wide operating temperature.

Although the present disclosure has been described above with reference to the preferred embodiment of the present disclosure, those skilled in the art will be able to understand that the present disclosure may be variously modified and changed without departing from the spirit and scope of the present disclosure as described in the claims below.

## Claims

1. A hydrogen detecting sensor comprising:
a substrate;
a heater layer formed on the substrate so as to generate heat;
a sensing element formed on a top face of the heater layer, wherein the sensing element includes a sensing layer, wherein the sensing layer has a structure in which two or more alloy layers are stacked, wherein each of the two or more alloy layers is made of an alloy of a catalyst metal and a transition metal, wherein an electrical resistance of the catalyst metal reversibly changes when the catalyst metal adsorbs hydrogen, wherein a ratio of a content of the transition metal to a content of the catalyst metal in each of the two or more alloy layers continuously changes based on a vertical level metal in each of the two or more alloy layers, wherein the sensing element measures an electrical resistance based on a hydrogen concentration; and
a compensation element formed on the top face of the heater layer so as to be spaced apart from the sensing element, wherein the compensation element includes:
a material having the same structure as the structure of the sensing layer; and
a protective layer covering the material layer so as to prevent an external substance from invading the material layer,
wherein the compensation element measures an electrical resistance based on temperature change.

2. The hydrogen detecting sensor of claim 1, wherein the sensing element further includes:
first and second electrodes in contact with the sensing layer and spaced apart from each other; and
an analysis circuit electrically connected to the first and second electrodes so as to measure change in the electrical resistance of the sensing layer based on the hydrogen concentration.

3. The hydrogen detecting sensor of claim 1, wherein the catalyst metal includes palladium or platinum, and the transition metal includes nickel or magnesium.

4. The hydrogen detecting sensor of claim 1, wherein in each of the alloy layers, the ratio of the content of the transition metal to the content of the catalyst metal in an area adjacent to each of a top face and a bottom face of each alloy layer is lower than the ratio at a vertical middle level between the top face and the bottom face.

5. The hydrogen detecting sensor of claim 4, wherein the ratio of the content of the transition metal to the content of the catalyst metal is highest at the vertical middle level in each of the alloy layers,
wherein the ratio gradually decreases as each of the alloy layers extends toward each of the top face and the bottom face.

6. The hydrogen detecting sensor of claim 1, wherein the heater layer is made of platinum (Pt), and is adhered to the substrate via an adhesive layer including titanium (Ti) or chromium (Cr).

7. The hydrogen detecting sensor of claim 1, wherein the compensation element further includes:
third and fourth electrodes in contact with the material layer coated with the protective layer and spaced apart from each other; and
an analysis circuit electrically connected to the third and fourth electrodes so as to measure change in the electrical resistance of the material layer based on the temperature change.

8. The hydrogen detecting sensor of claim 1, wherein the protective layer includes PTFE (polytetrafluoroethylene), PDMS (polydimethylsiloxane) or aluminum oxide (Al₂O₃).

9. A method for manufacturing a hydrogen detecting sensor, the method comprising:
depositing platinum on a substrate to form a heater layer;
disposing a plurality of transition metal layers and a plurality of catalyst metal layers on the top face of the heater layer such that the plurality of transition metal layers and the plurality of catalyst metal layers are alternately stacked on top of each other, wherein each of the catalyst metal layers is made of a catalyst metal whose an electrical resistance reversibly changes when the catalyst metal adsorbs hydrogen, wherein each of the plurality of transition metal layers is made of a transition metal suppressing phase transformation of the catalyst metal;
diffusing the transition metal into the catalyst metal layers to alloy the catalyst metal with the transition metal to form a hydrogen sensing layer made of the alloy, thereby manufacturing a sensing element including the sensing layer;
forming a material layer on the top face of the heater layer in the same manner as the formation manner of the hydrogen sensing layer, wherein the material layer is spaced apart from the hydrogen sensing layer; and
forming a protective layer covering an exposed surface of the material layer, thereby manufacturing a compensation element including the material layer and the protective layer.

10. The method of claim 9, wherein each of the catalyst metal layers is formed by performing a sputtering process of palladium or platinum,
wherein each of the transition metal layers is formed by performing a sputtering process of nickel or magnesium.

11. The method of claim 9, wherein each of the catalyst metal layers is formed to have a thickness in a range of 1 nm to 4 nm,
wherein each of the transition metal layers is formed to have a thickness of 0.1 to 0.5 times of the thickness of the catalyst metal layer.

12. The method of claim 11, wherein each of the transition metal layers is formed to have a thickness of 0.1 to 0.3 times of the thickness of the catalyst metal layer.

13. The method of claim 9, wherein the heater layer is formed by performing an e-beam deposition process of platinum,
wherein the heater layer is adhered to the substrate via an adhesive layer including titanium (Ti) or chromium (Cr).

14. The method of claim 9, wherein the protective layer is formed by performing an atomic layer deposition (ALD) process of aluminum oxide (Al₂O₃).

15. The method of claim 9, wherein the protective layer is formed by performing a deposition process or sputtering process of polytetrafluoroethylene (PTFE) or polydimethylsiloxane (PDMS).
